# EUROPEAN PATENT SPECIFICATION

(11) **EP 4 233 866 B1**
(45) Date of publication and mention of the grant of the patent: **28.10.2026**
(21) Application number: 21896891.5
(22) Date of filing: 19.11.2021
(51) Int. Cl.: A61K 31/675, A61K 31/506, A61K 31/44, A61K 31/4045, A61P 35/00

(54) **APPLICATION OF FOSTAMATINIB IN PREPARATION OF REAGENT FOR DOWN-REGULATING RUNX2 EXPRESSION**
ANWENDUNG VON FOSTAMATINIB BEI DER HERSTELLUNG EINES REAGENS ZUR HERUNTERREGULIERUNG DER RUNX2-EXPRESSION
APPLICATION DE FOSTAMATINIB DANS LA PRÉPARATION D'UN RÉACTIF POUR LA RÉGULATION NÉGATIVE DE L'EXPRESSION DE RUNX2

(30) Priority: 24.11.2020 CN 202011332972
(43) Date of publication of application: 30.08.2023
(73) Proprietor: Geneheal Biotechnology Co., Ltd., Guangzhou, Guangdong 510630 (CN)
(72) Inventor: PAN, Wuguang, Guangzhou, Guangdong 510630 (CN); ZHU, Wei, Guangzhou, Guangdong 510630 (CN)
(74) Representative: Santarelli
(86) International application number: PCT/CN2021/131753
(87) International publication number: WO 2022/111390

(56) References cited:
- WO-A1-2018/023113
- WO-A2-2018/005519
- CN-A- 105 849 110
- CN-A- 109 996 544
- JOSCHKA LORENZ ET AL: "From CLL to Multiple Myeloma - Spleen Tyrosine Kinase (SYK) influences multiple myeloma cell survival and migration", BRITISH JOURNAL OF HAEMATOLOGY, JOHN WILEY, HOBOKEN, USA, vol. 174, no. 6, 22 October 2015 (2015-10-22), pages 985 - 989, XP071121602, ISSN: 0007-1048, DOI: 10.1111/BJH.13825
- KIYOSHI YOSHIDA; CHIKAHISA HIGUCHI; AKIO NAKURA; HIDEKI YOSHIKAWA;: "Spleen tyrosine kinase suppresses osteoblastic differentiation through MAPK and PKC", BIOCHEMICAL AND BIOPHYSICAL RESEARCH COMMUNICATIONS, ELSEVIER, AMSTERDAM NL, vol. 411, no. 4, Amsterdam NL , pages 774 - 779, XP028382909, ISSN: 0006-291X, DOI: 10.1016/j.bbrc.2011.07.023
- WANG JIANAN, LIN WANG, SAIPENG CHEN, HUAHONG PENG, LONGFEI XIAO, DU E, YAN LIU, DONG LIN, YUZHUO WANG, YONG XU, KUO YANG: "PKMYT1 is associated with prostate cancer malignancy and may serve as a therapeutic target", GENE, vol. 744, 29 March 2020 (2020-03-29), XP055870445, DOI: 10.1016/j.gene.2020.144608
- SHINDE APARNA, HARDY SHANA D., KIM DONGWOOK, AKHAND SAEED SALEHIN, JOLLY MOHIT KUMAR, WANG WEN-HUNG, ANDERSON JOSHUA C., KHODADADI: "Spleen Tyrosine Kinase–Mediated Autophagy Is Required for Epithelial–Mesenchymal Plasticity and Metastasis in Breast Cancer", CANCER RESEARCH, AMERICAN ASSOCIATION FOR CANCER RESEARCH, US, vol. 79, no. 8, 15 April 2019 (2019-04-15), US , pages 1831 - 1843, XP055938072, ISSN: 0008-5472, DOI: 10.1158/0008-5472.CAN-18-2636
- SERAFIN VALENTINA, PORCÙ ELENA, CORTESE GIULIANA, MARIOTTO ELENA, VELTRI GIULIA, BRESOLIN SILVIA, BASSO GIUSEPPE, ACCORDI BENEDETT: "SYK Targeting Represents a Potential Therapeutic Option for Relapsed Resistant Pediatric ETV6-RUNX1 B-Acute Lymphoblastic Leukemia Patients", INTERNATIONAL JOURNAL OF MOLECULAR SCIENCES, vol. 20, no. 24, pages 6175, XP055938076, DOI: 10.3390/ijms20246175

## Description

### Technical Field

The present invention relates to new applications of compounds, in particular to the application of compounds in the preparation of down-regulating RUNX2 expression reagents, antitumor drugs, etc.

### Background of the Invention

Runx2 is a recognized as a transcription factor that plays an important role in the transformation of bone marrow mesenchymal stem cells into osteoblasts. In vitro and in vitro studies have shown that it can participate in bone metabolism, ectopic calcification of the cardiovascular system, abnormal development of teeth, tumor and organ fibrosis and other diseases through various signal transduction pathways such as bone morphogenetic protein/Smads, mitogen-activated protein kinase signaling pathway. The specific mechanism of action of Runx2 in the occurrence, development and prognosis of diseases has not been fully understood, and the treatment of clinical diseases with Runx2 as a target may become a research hotspot. Studies have shown that Runx2 is highly expressed in a variety of tumors, such as breast cancer, lung cancer, lung adenocarcinoma, prostate cancer, multiple myeloma, and malignant lobed tumors of the breast, which directly affects the progression of tumors. RUNX2 is overexpressed in melanoma cells and mediates their migration and invasion (Boregowda et al, Cancer Letters, 2014, 348(1-2):61-70).

Tumors seriously endanger people's health, and different tumors have different responses to the same drug. In order to improve the efficacy and reduce side effects, it has become a consensus of tumor treatment to formulate a reasonable medication scheme based on the phenotype of the tumor.

Fostamatinib is the prodrug of the active metabolite R406 of the spleen tyrosine kinase (SYK) inhibitor, DrugBank ID: DB12010, CAS number: 945745-48-2, has a variety of medical therapeutic effects, by developed by Rigel Pharmaceuticals of the United States, it is used for the treatment of thrombocytopenia, especially for adult patients with chronic immune thrombocytopenia (ITP) who have not been well relieved by previous treatment regimens. Fostamatinib has also been proposed as a therapeutic agent for inhibiting the proliferation of prostate cancer cells (Wang Jianan et al., "PKWYTI is associated with prostate cancer malignancy and may serve as a therapeutic target, " Gene, vol. 744, 29 March 2020). In addition, the use of fostamatinib for the treatment of cancer has been described in combination with a ROR-1 antagonist (WO 2018/005519 A2).

It is also known that some Syk inhibitors can stimulate mRNA expression of the osteoblastic marker Runx2 (Kiyoshi Yoshida et al: "Spleen tyrosine kinase suppresses osteoblastic differentiation through MAPK and PKC", Biochemical and Biophysical Research Communications, vol. 411, no. 4, pages 774-779) and the international patent application WO 2018/023113 A1 mentions that Syk inhibitors are known as anticancer agents. Furthermore, inhibition of Syk with R406, the active moiety of fostamatinib, was shown to significantly decrease the viability of multiple myeloma (MM) cell lines and to induce additional significant cytotoxicity when combined with bortezomib (Joschka Lorenz et al., "From CLL to Multiple Myeloma - Spleen Tyrosine Kinase (SYK) influences multiple myeloma cell survival and migration," British Journal of Haematology, vol. 174, no. 6, 22 October 2015, pp. 985-989).

Regorafenib, DrugBank ID: DB08896, CAS number: 755037-03-7, is a variety of small molecule inhibitors of intracellular kinases bound to cell membranes that are associated with normal cell function and pathological processes such as tumorigenesis, tumor angiogenesis, and maintenance of tumor microenvironment. In vitro biochemical or cellular analysis, ragorafenib or its main human active metabolites M-2 and M-5 inhibit RET, VEGFR1, VEGFR2, VEGFR3, KIT, PDGFR-alpha, PDGFR-beta, FGFR1, FGFR2, TIE2, DDR2, TrkA, Eph2A, RAF-1, BRAF, BRAFV600E at Regorafenib concentrations, SAPK2, PTK5 and Abl. In vivo models, ragorafenib showed antiangiogenic activity in rat tumor models and inhibition of tumor growth as well as antimetastatic activity in several mouse xenograft models, including some human colorectal cancers. Regorafenib has relatively large side effects, and its approved clinical use is for the treatment of patients with metastatic colorectal cancer who have previously received fluorouracil, oxaliplatin, and irinotecan-based chemotherapy, and who have previously received or are not candidates for anti-VEGF therapy and anti-EGFR therapy (RAS wild type); For the treatment of patients with locally advanced, unresectable or metastatic gastrointestinal stromal tumors who have previously received imatinib mesylate and sunitinib malate; For the treatment of hepatocellular carcinoma that has previously been treated with sorafenib.

Melatonin (MT) is one of the hormones secreted by the pineal gland of the brain, DrugBank ID: DB01065, CAS number: 73-31-4, also known as pineal gland, melatonin, melatonin. MT has strong neuroendocrine immunomodulatory activity and free radical scavenging antioxidant capacity.

### Technical Issues

It is of great importance to develop new applications for existing compounds.

### Technical Solution

### The present invention is defined in the appended set of claims.

The technical solution adopted in the present disclosure is as follows:
A first aspect of the present invention provides:
Use of fostamatinib and acceptable salts thereof as downregulating reagent of Runx2 expression in laboratory experimental applications outside human and animal body

In some embodiments, said salt is an acid or base addition salt of the compound.

In some embodiments, said acid is selected from a group consist of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid; the base is a hydroxide of the alkali metal, alkaline earth metal.

In some embodiments, the use is for experimental use.

A second aspect of the invention, providing:
A composition for use in the treatment of a tumor of breast cancer, lung cancer, lung adenocarcinoma, prostate cancer, multiple myeloma, or malignant phyllodes tumors of the breast, wherein the active ingredient of the composition is selected from Fostamatinib and acceptable salts thereof, and wherein the tumor expresses high levels of Runx2 which are significantly increased as compared to that of a normal tissue

In some embodiments, said composition include at least one extra known active ingredient with therapeutic effects on tumors.

In some embodiments, the salt is an acid or base addition salt of the compound.

In some embodiments, said acid is selected from a group consist of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid; the base is a hydroxide of the alkali metal, alkaline earth metal.

### Beneficial Effect

Through computational studies and disease models, the inventors have shown that:
Fostamatinib, Regorafenib and melatonin can significantly down-regulate the expression of Runx2, has a significant targeted therapeutic effect on cancers with abnormally high expression of Runx2, and is an efficient Runx2 expression down-regulating reagent, especially for the laboratory use. Regorafenib and melatonin are not part of the present invention and related data is disclosed for information purposes only.

By use of one of Fostamatinib, Regorafenib, and melatonin, or in combination, or in combination with a known antitumor drug, efficacy can be effectively improved and/or side effects be reduced.

Acceptable salts of Fostamatinib, Regorafenib or melatonin, in vivo or cellular environment, can produce the same or similar effects as Fostamatinib, Regorafenib or melatonin, and also have corresponding curative effects or effects.

### Brief description of the Drawings

Figure 1 shows the expression level of RUNX2 protein after Fostamatinib acts on lung cancer cells A549 and lung cancer cell H1299 for 8 h, respectively.
Figure 2 shows the expression level of RUNX2 mRNA after Fostamatinib acts on lung cancer cells A549 and lung cancer cell H1299 for 8 h, respectively.
Figure 3 shows the expression level of RUNX2 protein after Fostamatinib acts on lung cancer cells A549 and lung cancer cell H1299 for 6 h, respectively.
Figure 4 shows the expression level of RUNX2 mRNA after Fostamatinib acts on lung cancer cells A549 and lung cancer cell H1299 for 6 h, respectively.
Figure 5 shows the expression level of RUNX2 protein after treatment of BEL-7402 for 6 h in hepatoma cells with different concentrations of Fostamatinib.
Figure 6 shows the Effect of fostamatinib intraperitoneal injection on Runx2 mRNA expression levels in mice.

### Detailed Description of Embodiments

As used herein, the term "high expression" has the meaning well known in the art, and refers to the expression which is significantly increased as compared to that of a normal tissue. In general, the expression is 1.5 times, 2 times, 3 times, 5 times or more than that of normal tissues.

Pharmaceutically acceptable salts of the compounds can be synthesized from parent compounds by conventional chemical methods, such as those in Pharmaceutical Salts: Properties, Selection, and Use, P. Heinrich Stahl (Editor), Camille G. Wermuth (Editor), ISBN: 3-90639-026-8, Hardcover, 388 pages, August 2002. In general, these salts can be prepared by the reaction of the free base and acid of the compound in water or an organic solvent or a mixture of the two; Typically, non-aqueous media such as ether, ethyl acetate, ethanol, isopropanol or acetonitrile are used.

Acid addition salts can be made by various acids (inorganic and organic acids). Embodiments of acid addition salts include a salt made by a compounds reacted with an acid, said acid is selected from a group consist of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid.

Through computational studies and preliminary experimental studies of related disease models, the inventors have confirmed that:
1) Fostamatinib, Regorafenib and melatonin can significantly down-regulate the expression of Runx2, has a significant targeted therapeutic effect on cancers with abnormally high expression of Runx2, and is an efficient Runx2 expression down-regulating reagent, especially for the laboratory use.
2) By use of one of Fostamatinib, Regorafenib, and melatonin, or in combination, or in combination with a known antitumor drug, efficacy can be effectively improved and/or side effects be reduced.
3) Acceptable salts of Fostamatinib, Regorafenib or melatonin, in vivo or cellular environment, can produce the same or similar effects as Fostamatinib, Regorafenib or melatonin, and also have corresponding curative effects or effects.

The preliminary experiment is as follows:

### Effects of fostamatinib on protein and mRNA expression of Runx2 in tumor cells

Cells are seeded in medium dishes (6cm), cultured overnight, when the cell confluency is 80%, the original medium is discarded, PBS rinses the cells, and then replaced with Fostamatinib containing a final concentration of 10uM or other concentrations, after 6~12 hours, the cells are harvested, RNA and protein liquid are extracted, and the protein and mRNA expression changes of RUNX2 are detected by Western blot and qPCR experiments, respectively.

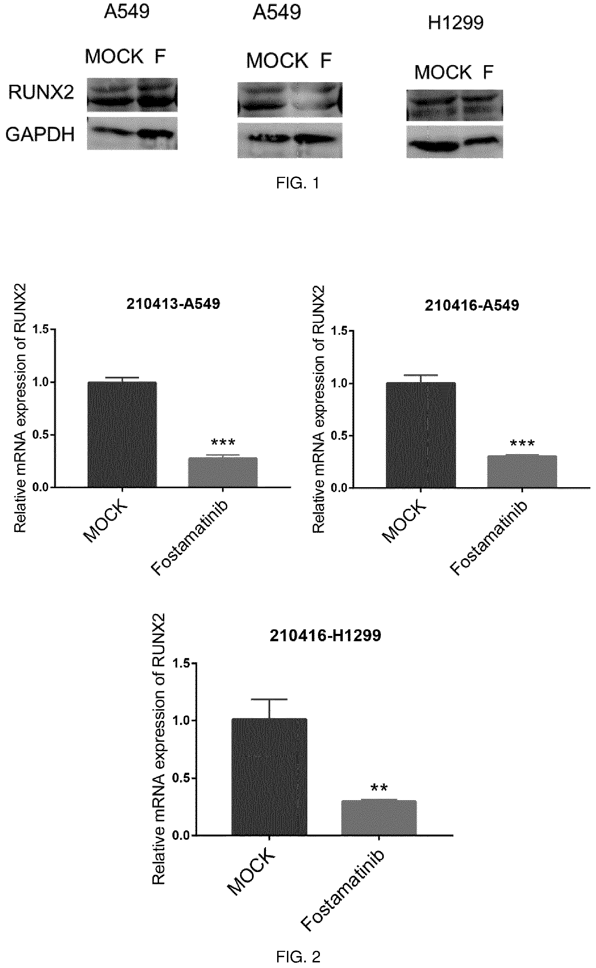

The experimental results are shown in Figure 1~5, wherein:
Figure 1 shows that after treated with Fostamatinib for 8 hours respectively, the protein expression level of RUNX2 of lung cancer cells A549 and lung cancer cell H1299 tends to decrease (in figure 1, F represents Fostamatinib).
Figure 2 shows that after treated with Fostamatinib for 8 hours respectively, the mRNA expression level of RUNX2 of lung cancer cells A549 and lung cancer cell H1299 decreases significantly.

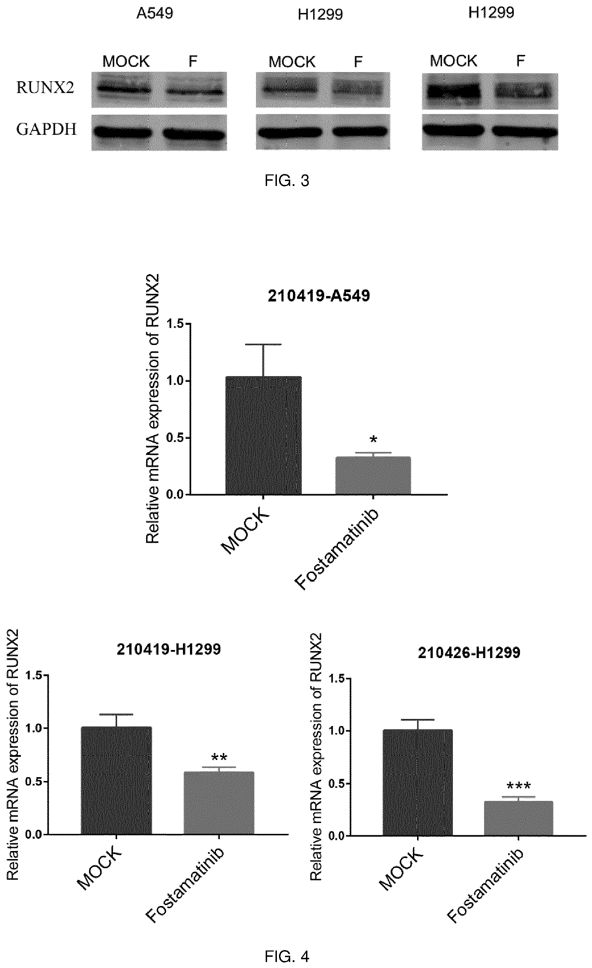

Figure 3 shows that after treated with Fostamatinib for 6 hours respectively, the protein expression level of RUNX2 of lung cancer cells A549 and lung cancer cell H1299 down-regulated a certain trend (in the figure, F indicates Fostamatinib, the antibody has been changed).
Figure 4 shows that after Fostamatinib acts on lung cancer cells A549 and lung cancer cells H1299 for 6 hours respectively, the mRNA level of RUNX2 is significantly reduced.

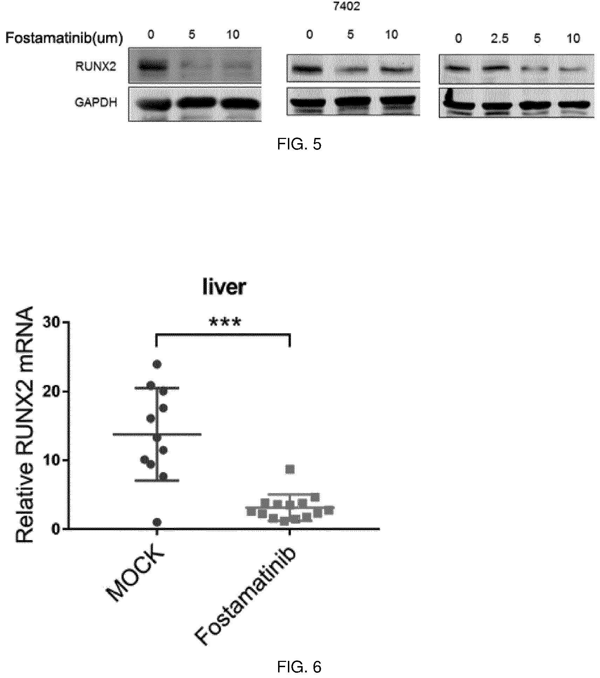

Fig. 5 shows that after treating hepatoma cells BEL-7402 with 0, 2.5, 5, and 10 uM Fostamatinib for 6 h, the proteins level of hepatoma cells is significantly reduced and dose-dependent.

The above experimental results show that Fostamatinib can well inhibit the expression of mRNA of RUNX2 and has anti-tumor effects.

### Animal testing (intraperitoneal injection)

Drug: Fostamatinib MCE(HY-13038A)
Mice: 8-week-old C57BL / 6 male mice
Concentration of administration: 40mg/kg
Mode of administration: intraperitoneal injection
Experimental steps: mice are randomly divided into two groups, experimental group (fostamatinib) and control group (mock), before daily experiments, a certain amount of fostamatinib is accurately weighted and dissolve in 0.5% CMC (nitromethyl cellulose), fully pipetted into a uniform suspension, the concentration of 4mg/ml, and ready for use. According to the injection volume of 100ul/10g per mouse, the intraperitoneal injection was carried out for 4 consecutive days. The mouse liver tissue was collected 48 hours after the last injection, RNA was extracted, and the mRNA expression level of RUNX2 was detected by real-time fluorescence quantitative PCR (qPCR). The experiment results are shown in Figure 6. It's clear that fostamatinib can significantly reduce the mRNA expression level of RUNX2 and has anti-tumor effects.

## Claims

1. Use of fostamatinib and acceptable salts thereof as downregulating reagent of Runx2 expression in laboratory experimental applications outside human and animal body.

2. The use of claim 1, wherein said salts are acid or base addition salts of the compounds; preferably, the acid is selected from a group consist of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid; the base is a hydroxide of the alkali metal, alkaline earth metal.

3. Composition for use in the treatment of a tumor of breast cancer, lung cancer, lung adenocarcinoma, prostate cancer, multiple myeloma, or malignant phyllodes tumors of the breast, wherein the active ingredient of the composition is selected from Fostamatinib and acceptable salts thereof, and wherein the tumor expresses high levels of Runx2 which are significantly increased as compared to that of a normal tissue.

4. Composition for use according to claim 3, wherein the composition includes at least one extra known active ingredient with therapeutic effects on tumors.

5. Composition for use according to claim 3, wherein said salts are acid or base addition salts of the compounds; preferably, the acid is selected from a group consisting of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid; the base is a hydroxide of the alkali metal, alkaline earth metal.

## Patentansprüche

1. . Verwendung von Fostamatinib und akzeptablen Salzen davon als Herabregulierungsreagenz der Runx2-Expression in experimentellen Laboranwendungen außerhalb des menschlichen und tierischen Körpers.

2. . Verwendung nach Anspruch 1, wobei die Salze Säure- oder Basenzusatzsalze der Verbindungen sind; die Säure bevorzugt aus einer Gruppe ausgewählt wird, die aus Essigsäure, 2,2-Dichloressigsäure, Adipinsäure, Alginsäure, Ascorbinsäure, L-Asparaginsäure, Benzensulfonsäure, Benzoesäure, 4-Acetylaminobenzoesäure, Buttersäure, (+)-Camphersäure, Camphersulfonsäure, (+)-(1S)-Kampher-10-Sulfonsäure, Caprinsäure, Hexansäure, Oktansäure, Zimtsäure, Zitronensäure, Cyclaminsäure, Dodecylschwefelsäure, Ethan-1,2-Disulfonsäure, Ethansulfonsäure, 2-Hydroxyethansulfonsäure, Ameisensäure, Fumarsäure, Galaktonsäure, Gentisinsäure, Glucoheptonsäure, D-Gluconsäure, Glucuronsäure, Glutaminsäure, α-Ketoglutarsäure, Glykolsäure, Hippursäure, Bromwasserstoffsäure, Salzsäure, Jodwasserstoffsäure, Isethionsäure, (+)-L-Milchsäure, (±)-DL-Milchsäure, Laktobionsäure, Malsäure, Apfelsäure, (-)-L-Apfelsäure, Malonsäure, (±)-DL-Mandelsäure, Methansulfonsäure, Naphthalin-2-Sulfonsäure, Naphthalin-1,5-Disulfonsäure, 1-Hydroxyl-2-Naphthoinsäure, Nikotinsäure, Salpetersäure, Ölsäure, Orotsäure, Oxalsäure, Palmitinsäure, Pamoinsäure, Phosphorsäure, Propionsäure, L-Pyroglutaminsäure, Salicylsäure, 4-Aminosalicylsäure, Sebacinsäure, Stearinsäure, Succinsäure, Schwefelsäure, Gerbsäure, (+)-L-Weinsäure, Sulfocyansäure, p-Toluensulfonsäure, Undecylensäure, Pentansäure und acylierter Aminosäure besteht; wobei die Basis ein Hydroxid des Alkalimetalls, Erdalkalimetall ist.

3. . Zusammensetzung zur Verwendung bei der Behandlung eines Tumors von Brustkrebs, Lungenkrebs, Adenokarzinom der Lunge, Prostatakrebs, Multiplem Myelom oder bösartigen Phyllodentumoren der Brust, wobei der Wirkstoff der Zusammensetzung aus Fostamatinib und akzeptablen Salzen davon ausgewählt ist, und wobei der Tumor hohe Runx2-Spiegel exprimiert, die im Vergleich zu jenen eines normalen Gewebes signifikant erhöht sind.

4. . Zusammensetzung zur Verwendung nach Anspruch 3, wobei die Zusammensetzung mindestens einen zusätzlichen bekannten Wirkstoff mit therapeutischen Wirkungen auf Tumoren umfasst.

5. . Zusammensetzung zur Verwendung nach Anspruch 3, wobei die Salze Säure- oder Basenzusatzsalze der Verbindungen sind; die Säure bevorzugt aus einer Gruppe ausgewählt ist, die aus Essigsäure, 2,2-Dichloressigsäure, Adipinsäure, Alginsäure, Ascorbinsäure, L-Asparaginsäure, Benzensulfonsäure, Benzoesäure, 4-Acetylaminobenzoesäure, Buttersäure, (+)-Camphersäure, Camphersulfonsäure, (+)-(1S)-Campher-10-Sulfonsäure, Caprinsäure, Hexansäure, Oktansäure, Zimtsäure, Zitronensäure, Cyclaminsäure, Dodecylschwefelsäure, Ethan-1,2-Disulfonsäure, Ethansulfonsäure, 2-Hydroxyethansulfonsäure, Ameisensäure, Fumarsäure, Galaktonsäure, Gentisinsäure, Glucoheptonsäure, D-Gluconsäure, Glucuronsäure, Glutaminsäure, α-Ketoglutarsäure, Glykolsäure, Hippursäure, Bromwasserstoffsäure, Salzsäure, Salzsäure, Isethionsäure, (+)-L-Milchsäure, (±)-DL-Milchsäure, Laktobionsäure, Maleinsäure, Apfelsäure, (-)-L-Apfelsäure, Malonsäure, (±)-DL-Mandelsäure, Methansulfonsäure, Naphthalin-2-Sulfonsäure, Naphthalin-1,5-Disulfonsäure, 1-Hydroxyl-2-Naphthoinsäure, Nikotinsäure, Salpetersäure, Ölsäure, Orotsäure, Oxalsäure, Palmitinsäure, Pamoinsäure, Phosphorsäure, Propionsäure, L-Pyroglutaminsäure, Salicylsäure, 4-Aminosalicylsäure, Sebacinsäure, Stearinsäure, Succinsäure, Schwefelsäure, Gerbsäure, (+)-L-Weinsäure, Sulfocyansäure, p-Toluensulfonsäure, Undecylensäure, Pentansäure und acylierter Aminosäure besteht; wobei die Basis ein Hydroxid des Alkalimetalls, Erdalkalimetall ist.

## Revendications

1. Utilisation du fostamatinib et de ses sels acceptables comme réactif de régulation négative de l'expression de Runx2 dans des applications expérimentales en laboratoire en dehors du corps humain et animal.

2. Utilisation selon la revendication 1, dans laquelle lesdits sels sont des sels d'addition acides ou basiques des composés ; de préférence, l'acide est sélectionné dans un groupe comprenant l'acide acétique, l'acide 2,2-dichloroacétique, l'acide adipique, l'acide alginique, l'acide ascorbique, l'acide L-aspartique, l'acide benzènesulfonique, l'acide benzoïque, l'acide 4-acétylaminobenzoïque, l'acide butyrique, l'acide (+)-camphorique, l'acide camphorsulfonique, l'acide (+)-(1S)-camphor-10-sulfonique, l'acide caprique, l'acide hexanoïque, l'acide octanoïque, l'acide cinnamique, l'acide citrique, l'acide cyclamique, l'acide dodécylsulfurique, l'acide éthane-1,2-disulfonique, l'acide éthanesulfonique, l'acide 2-hydroxyéthanesulfonique, l'acide formique, l'acide fumarique, l'acide galactonique, l'acide gentisique, l'acide glucoheptonique, l'acide D-gluconique, l'acide glucuronique, l'acide glutamique, l'acide α-cétoglutarique, l'acide glycolique, l'acide hippurique, l'acide bromhydrique, l'acide chlorhydrique, l'acide iodhydrique, l'acide iséthionique, l'acide (+)-L-lactique, l'acide (±)-DL-lactique, l'acide lactobionique, l'acide maléique, l'acide malique, l'acide (-)-L-malique, l'acide malonique, l'acide (±)-DL-mandélique, l'acide méthanesulfonique, l'acide naphtalène-2-sulfonique, l'acide naphtalène-1,5-disulfonique, l'acide 1-hydroxy-2-naphtoïque, l'acide nicotinique, l'acide nitrique, l'acide oléique, l'acide orotique, l'acide oxalique, l'acide palmitique, l'acide pamoïque, l'acide phosphorique, l'acide propionique, l'acide L-pyroglutamique, l'acide salicylique, l'acide 4-aminosalicylique, l'acide sébacique, l'acide stéarique, l'acide succinique, l'acide sulfurique, l'acide tannique, l'acide (+)-L-tartarique, l'acide sulfocyanique, l'acide p-toluenesulfonique, l'acide undécylénique, l'acide pentanoïque et un acide aminé acylé ; la base est une hydroxyde de métal alcalin ou de métal alcalino-terreux.

3. Composition à utiliser dans le traitement d'une tumeur de cancer du sein, cancer du poumon, adénocarcinome pulmonaire, cancer de la prostate, myélome multiple ou tumeurs phyllodes malignes du sein, dans laquelle le principe actif de la composition est choisi parmi le fostamatinib et ses sels acceptables, et dans laquelle la tumeur exprime des niveaux élevés de Runx2 significativement accrus par rapport à ceux d'un tissu normal.

4. Composition à utiliser selon la revendication 3, dans laquelle la composition comprend au moins un ingrédient actif supplémentaire connu ayant des effets thérapeutiques sur les tumeurs.

5. Composition à utiliser selon la revendication 3, dans laquelle lesdits sels sont des sels d'addition acides ou basiques des composés ; de préférence, l'acide est choisi dans un groupe comprenant l'acide acétique, l'acide 2,2-dichloroacétique, l'acide adipique, l'acide alginique, l'acide ascorbique, l'acide L-aspartique, l'acide benzènesulfonique, l'acide benzoïque, l'acide 4-acétylaminobenzoïque, l'acide butyrique, l'acide (+)-camphorique, l'acide camphorésulfonique, l'acide (+)-(1S)-camphor-10-sulfonique, l'acide caprique, l'acide hexanoïque, l'acide octanoïque, l'acide cinnamique, l'acide citrique, l'acide cyclamique, l'acide dodécylsulfurique, l'acide éthane-1,2-disulfonique, l'acide éthanesulfonique, l'acide 2-hydroxyéthanesulfonique, l'acide formique, l'acide fumarique, l'acide galactonique, l'acide gentisique, l'acide glucoheptonique, l'acide D-gluconique, l'acide glucuronique, l'acide glutamique, l'acide α-cétoglutarique, l'acide glycolique, l'acide hippurique, l'acide bromhydrique, l'acide chlorhydrique, l'acide iodhydrique, l'acide iséthionique, l'acide (+)-L-lactique, l'acide (±)-DL-lactique, l'acide lactobionique, l'acide maléique, l'acide malique, l'acide (-)-L-malique, l'acide malonique, l'acide (±)-DL-mandélique, l'acide méthanesulfonique, l'acide naphtalène-2-sulfonique, l'acide naphtalène-1,5-disulfonique, l'acide 1-hydroxy-2-naphtoïque, l'acide nicotinique, l'acide nitrique, l'acide oléique, l'acide orotique, l'acide oxalique, l'acide palmitique, l'acide pamoïque, l'acide phosphorique, l'acide propionique, l'acide L-pyroglutamique, l'acide salicylique, l'acide 4-aminosalicylique, l'acide sébacique, l'acide stéarique, l'acide succinique, l'acide sulfurique, l'acide tannique, l'acide (+)-L-tartrique, l'acide sulfocyanique, l'acide p-toluenesulfonique, l'acide undécylénique, l'acide pentanoïque, et un acide aminé acylé ; la base est un hydroxyde de métal alcalin ou alcalino-terreux.
